(19) Europäisches Patentamt
European Patent Office
Office européen des brevets

(11) Veröffentlichungsnummer: **0 133 960**
**A2**

(12) **EUROPÄISCHE PATENTANMELDUNG**

(21) Anmeldenummer: **84108881.8**

(22) Anmeldetag: **26.07.84**

(51) Int. Cl.[4]: **C 07 C 69/88,** C 07 C 67/30

(30) Priorität: **04.08.83 CH 4231/83**

(43) Veröffentlichungstag der Anmeldung: **13.03.85 Patentblatt 85/11**

(84) Benannte Vertragsstaaten: **CH DE FR GB LI NL**

(71) Anmelder: **Roure Bertrand Dupont Société Anonyme, 55, Voie des Bans, F-95102 Argenteuil (FR)**

(72) Erfinder: **Pelerin, Gérard, Chemin de Stramousse, F-06820 Cabris (FR)**

(74) Vertreter: **Lederer, Franz, Dr. et al, Patentanwälte Lederer, Meyer Lucile-Grahn-Strasse 22, D-8000 München 80 (DE)**

(54) **Verfahren zur Herstellung von Estern substituierter Benzoesäuren.**

(57) Es wird ein Verfahren zur Herstellung von Estern der Formel

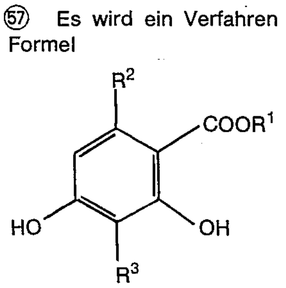

I

worin $R^1$ für Methyl oder Äthyl steht und
$R^2$ und $R^3$, unabhängig voneinander, Wasserstoff oder $C_{1-4}$-Alkyl darstellen,
beschrieben. Das Verfahren ist dadurch gekennzeichnet, daß man einen Diketoester der Formel

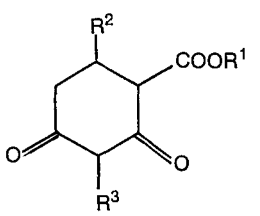

II

worin $R^1$ bis $R^3$ obige Bedeutung besitzen, mit der Kombination von

$CuX_2$ und $MeX_n$ III

worin Me ein Alkali- oder Edelalkalimetallion und
X Halogen bedeutet, und n der Wertigkeit des Metallions entspricht, behandelt.

EP 0 133 960 A2

SOCIETE ANONYME ROURE BERTRAND DUPONT Argenteuil (France)

# Verfahren zur Herstellung von Estern substituierter Benzoesäuren

1 Die Erfindung betrifft ein Verfahren zur Herstellung der, grösstenteils bekannten Ester der allgemeinen Formel

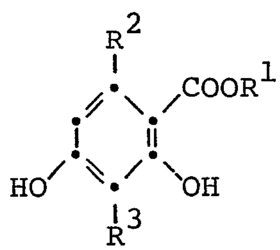

I

worin $R^1$ für Methyl oder Aethyl steht und $R^2$ und $R^3$, unabhängig voneinander, Wasserstoff oder $C_{1-4}$-Alkyl darstellen.

Das Verfahren ist dadurch gekennzeichnet, dass man einen Diketoester der Formel

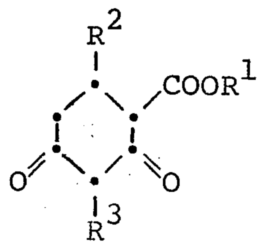

II

worin $R^1$ bis $R^3$ obige Bedeutung besitzen, mit der Kombination von

$CuX_2$ und $MeX_n$ III

worin Me ein Alkali- oder Erdalkalimetallion und X Halogen bedeutet, und n der Wertigkeit des Metallions entspricht,

behandelt.

Im Vordergrund des Interesses stehen die Ester I mit

$R^1$ = Methyl oder Aethyl und $R^2$ = $R^3$ = Methyl,
$R^1$ = Methyl oder Aethyl und $R^2$ = Methyl und $R^3$ = H.

Besonders bevorzugt ist die Verbindung mit $R^1$ = Methyl und $R^2$ = $R^3$ = Methyl.

Die Verbindungen I stellen bekannte Riechstoffe dar, siehe z.B. US-PS 3,634,491 und US-PS 3,944,596.

Die erfindungsgemässe Reaktion wird zweckmässigerweise in einem Lösungsmittel, vorzugsweise einem polaren Lösungsmittel durchgeführt. Als polare Lösungsmittel sind insbesondere die polaren aprotischen Lösungsmittel bevorzugt, also z.B. Dimethylformamid, Acetonitril oder Aether, insbesondere Diisopropyläther. Aber auch unpolare aprotische Lösungsmittel, wie chlorierte Kohlenwasserstoffe, z.B. Dichloräthan, oder polare protische Lösungsmittel, wie Alkohole, kommen in Frage.

X steht definitionsgemäss für Halogen, wobei alle Halogene, d.h. F, Cl, Br, I, in Frage kommen. Me steht definitionsgemäss für ein Alkali- oder Erdalkalimetallion, wobei sämtliche dieser Metalle, insbesondere Li, Na, K und Ca, Sr, Ba, in Frage kommen. Das Verhältnis von Cu zu Me in der Verbindung III ist zweckmässigerweise 1-5:1, vorzugsweise 1-2:1. Das Verhältnis von Verbindung II zu Verbindung III (auf Cu bezogen) beträgt zweckmässigerweise 0,1-0,5:1, vorzugsweise ist es 0,4-0,5:1.

Das erfindungsgemässe Verfahren wird zweckmässigerweise in einem Temperaturbereich von ca. 50 - ca. 120°C durchgeführt; der bevorzugte Temperaturbereich ist der von ca. 55 - ca. 80°C.

Die Herstellung der Ester I kann allgemein wie folgt durchgeführt werden:

Der Ketoester II, das Kupferhalogenid und das Halogenid $MeX_n$ werden als Suspension in das Lösungsmittel gegeben. Man erhitzt nun auf ca. 50 - 120°C und verfolgt den Reaktionsablauf chromatographisch, z.B. dünnschichtchromatographisch. Nach beendigter Reaktion wird das Reaktionsgemisch abgekühlt. Man behandelt die Lösung mit einem Ueberschuss einer Mineralsäure, vorzugsweise einer beispielsweise 10%-igen wässrigen Lösung von HCl. Im Falle der Wahl eines mit Wasser gänzlich mischbaren Lösungsmittels wie DMF oder Acetonitril, etc., nimmt man das Reaktionsgemisch in der Folge mit einem wasserunlöslichen Lösungsmittel auf. Beispiele sind 1,2-Dichloräthan, Diisopropyläther, etc. Hierauf trennt man die organische Schicht wieder von der wässrigen Phase. Hierauf extrahiert man die letztere. Die organischen Phasen werden mit Wasser neutral gewaschen. Das Lösungsmittel wird abdestilliert, das rohe Reaktionsprodukt I wird gereinigt, beispielsweise durch Chromatographie oder Kristallisieren, Sublimation etc.

Die Umsetzung von Diketoestern der Formel II zu Estern der Formel I mittels einer Zweistufenreaktion unter Verwendung von $CuBr_2$ in der ersten Stufe wird von R.S. Marmor in J. Org. Chem. 37, (1972), 2901 seq. beschrieben. Die auf diese Weise erzielten Ausbeuten betrugen jedoch nur 41-43%.

Weiterhin beschrieben Pfau et al. in Helv. Chim. Acta 16, (1933), 282 seq. die Umsetzung II→I unter Verwendung von $FeCl_3$. Die Ausbeute wird als mager bezeichnet. Es ist deshalb höchst überraschend, dass unter Verwendung von $CuX_2$ + $MeX_n$ gemäss dem vorliegenden Einstufenverfahren die Aus-

0133960

beute an I ausgehend von II so viel höher ist, nämlich in den meisten Fällen über 70%.

Beispiel

In ein mit Rührer, Thermometer und Rückflusskühler versehenes Reaktionsgefäss gibt man 99g (0,5 Mol) 3,6-Dimethyl-cyclohexan-2,4-dion-methylcarboxylat, 500 ml Acetonitril, 170,5 g $CuCl_2 \cdot 2H_2O$ (1 Mol) und 50,8g (0,25 Mol) $MgCl_2 \cdot 6H_2O$. Man erhitzt das Reaktionsgemisch 8 Stunden auf Rückflusstemperatur und stellt nach dieser Zeit das gänzliche Verschwinden des Dions fest. Man nimmt in 250 ml Diisopropyläther auf, wäscht mit 250 ml 10%-iger HCl und extrahiert die wässrige, dekantierte Phase 2 mal mit je 250 mlDiisopropyläther. Die vereinigten organischen Phasen werden 2 mal mit je 250 ml 10%-iger Salzsäure, hierauf 3 mal mit je 250 ml Wasser gewaschen. Die Lösungsmittel werden abdestilliert. Das erhaltene rohe Dihydroxyderivat wird zunächst aus 250 ml Toluol, hierauf 2 mal aus 50%-igem Methanol umkristallisiert. Die Umkristallisation geschieht unter Stickstoff. Man erhält 74,5 g (Ausbeute 76%) ockergelbe Kristalle von β-Orcin-methylcarboxylat. Smp: 143 - 144°C.
Identifikation: Mittels IR- und MNR-Daten wird der Verbindung die eindeutige Struktur

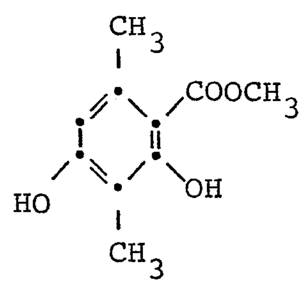

zugeordnet.

Auf analoge Weise stellt man aus dem 6-Methyl-cyclohexan-2,4-dion-methylcarboxylat das dem obigen I entsprechende Monomethylderivat her. Aus dem 6-Methyl-cyclohexan-2,4-dion-äthylcarboxylat wird das Orcin-äthylcarboxylat gewonnen.

Variation von Reaktionsparametern:

| Lösungsmittel | Kombination III | Ausbeute* |
|---|---|---|
| DMF | $CuCl_2 \cdot 2H_2O$ - $CaCl_2$ | 55% |
| $CH_3CN$ | $CuCl_2 \cdot 2H_2O$ - $BaCl_2 \cdot 2H_2O$ | 86% |
| $CH_3CN$ | $CuCl_2 \cdot 2H_2O$ - Na I $\cdot 2H_2O$ | 81% |
| $CH_3CN$ | $CuCl_2 \cdot 2H_2O$ - NaCl | 76% |
| $CH_3CN$ | $CuBr_2$ - $CaCl_2$ | 58% |
| 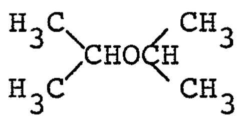 | $CuBr_2$ - LiBr | 76% |

* Herstellung des β-Orcin-methylcarboxylats.

 

Patentansprüche

1. Verfahren zur Herstellung von Estern der Formel

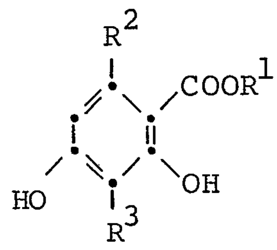

I

worin $R^1$ für Methyl oder Aethyl steht und $R^2$ und $R^3$, unabhängig voneinander, Wasserstoff oder $C_{1-4}$-Alkyl darstellen,

dadurch gekennzeichnet, dass man einen Diketoester der Formel

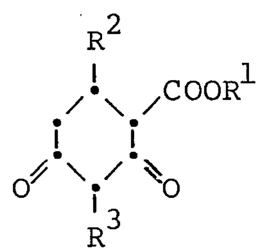

II

worin $R^1$ bis $R^3$ obige Bedeutung besitzen,

mit der Kombination von

$CuX_2$ und $MeX_n$ III

worin Me ein Alkali- oder Erdalkalimetallion, X Halogen und n der Wertigkeit des Metallions entspricht,

behandelt.

2. Verfahren nach Anspruch 1, dadurch gekennzeichnet, dass $R^1$ = Methyl oder Aethyl und $R^2$=$R^3$=Methyl oder $R^1$= Methyl oder Aethyl, $R^2$=Methyl und $R^3$=Wasserstoff darstellen.

3. Verfahren nach Anspruch 1 oder Anspruch 2, dadurch gekennzeichnet, dass man in einem polaren, insbesondere polaren aprotischen Lösungsmittel arbeitet.

4. Verfahren nach einem der Ansprüche 1 bis 3, dadurch gekennzeichnet, dass man in einem Aether arbeitet.

5. Verfahren nach Anspruch 4, dadurch gekennzeichnet, dass man in Diisopropyläther arbeitet.

6. Verfahren nach einem der Ansprüche 1 bis 5, dadurch gekennzeichnet, dass X für Chlor steht.

7. Verfahren nach einem der Ansprüche 1 bis 5, dadurch gekennzeichnet, dass Me für Calcium steht.

8. Verfahren nach einem der Ansprüche 1 bis 5, dadurch gekennzeichnet, dass das Verhältnis von Cu zu Me 1 - 5 : 1 ist.

9. Verfahren nach Anspruch 8, dadurch gekennzeichnet, dass Cu zu Me 1 - 2 : 1 ist.

10. Verfahren nach Anspruch 9, dadurch gekennzeichnet, dass das Verhältnis von II:III (auf Cu bezogen)=0,1-0,5:1 ist.

11. Verfahren nach Anspruch 10, dadurch gekennzeichnet, dass das Verhältnis von II : III = 0,4-0,5 : 1 ist.

12. Verfahren nach Anspruch 11, dadurch gekennzeichnet, dass in einem Temperaturbereich von ca. 50-ca. 120°C gearbeitet wird.

13. Verfahren nach Anspruch 12, dadurch gekennzeichnet, dass in einem Temperaturbereich von ca. 55-ca. 80°C gearbeitet wird.

* * *